# EUROPEAN PATENT APPLICATION

(11) **EP 4 675 633 A1**
(43) Date of publication of application: **07.01.2026**
(21) Application number: 24315318.6
(22) Date of filing: 02.07.2024
(51) Int. Cl.: G16H 20/40, G16H 30/20, G16H 30/40, G16H 40/63, G16H 50/20, G16H 50/30, G16H 50/70

(54) **A SYSTEM FOR AUTOMATICALLY ASSESSING A RISK OF PARAVALVULAR LEAKS**

(71) Applicant: Caranx Medical, 75013 Paris (FR)
(72) Inventor: WEI, Wen, 06410 Biot (FR); CERRUTI, Giulio, 06700 Saint-Laurent-Du-Var (FR); BERTHET-RAYNE, Pierre, 06800 Cagnes-sur-Mer (FR)
(74) Representative: Hepp Wenger Ryffel AG

(57) **Abstract**

The present invention relates to a system, a method, a computer program and a computer program product for automatically assessing a risk of paravalvular leaks after a clinical intervention for implanting a prosthetic cardiac valve in a patient according to the independent claims. A system (100) for automatically assessing a risk of paravalvular leaks after a clinical intervention for implanting a prosthetic cardiac valve in a patient comprises an input interface (101) for obtaining data and a computing unit (102). The computing unit (102) is adapted to obtain first input data (201) acquired before the start of the clinical intervention via the input interface (101), the first input data comprising image data of a patient and comprising image data of a region of interest including the aortic root, preferably CT image data. The computing unit (102) is preferably adapted to pre-analyse the first input data. The computing unit (102) is adapted to classify the patient into one of two main classes of likelihood of paravalvular leaks on basis of the first input data.

## Description

The present invention relates to a system, a method, a computer program and a computer program product for automatically assessing a risk of paravalvular leaks after a clinical intervention for implanting a prosthetic cardiac valve in a patient according to the independent claims.

It is known to replace aortic valves, mitral valves, tricuspid valves or pulmonary valves or an artificial heart valve by various valve replacement procedures. Current aortic valve replacement approaches include closed heart surgery. Trans-catheter aortic valve implantation (TAVI) or trans-catheter aortic valve replacement (TAVR) is a minimally invasive procedure for treating a failing aortic valve.

A valve implant (e.g. a bioprosthetic valve made of e.g. porcine pericardium sutured on a metal stent) typically is crimped inside a catheter. The catheter is inserted, for example, via the femoral artery and is pushed upstream along the aorta up to the diseased native valve, within which the valve implant is deployed e.g. via balloon expansion or self expansion. Main complications during implantation are vascular injury, stroke, cardiac injury, aortic regurgitation, cardiac conduction abnormalities and valve misplacement.

Paravalvular regurgitation is an important complication that has been shown to be associated with increased mortality for both balloon-expandable and self-expanding transcatheter heart valves.

Paravalvular leaks are believed to depend on multiple factors. Complex interactions between the native aortic root, the selected prosthesis, fast moving blood etc. complicate the accuracy of a risk assessment.

Corrective procedures such as balloon post-dilation, valve in valve deposition or closure of the leak with a plug, might be necessary.

In "Assessment of Paravalvular Regurgitation Following TAVR" (Pibarot et al, JACC: Cardiovascular Imaging, Vol. 8, No. 3, 2015) four methods for assessing paravalvular leaks are disclosed: cineangiography, hemodynamics, echocardiography and CMR. Also, an algorithm for quantification of aortic regurgitation and for a decision if corrective procedures have to be taken is proposed.

There is also an interest to predict the outcome of a clinical intervention prior to or during the clinical intervention.

Traditionally, risk assessments are carried out by a clinician to determine the health of a subject, and the likelihood of the subject being at risk of circulatory (for example, cardiovascular) diseases. These risk assessments take into account risk factors such as gender, age, smoking status, cholesterol levels, diabetes status, and family history. Additionally, images of circulatory systems of subjects may be taken, in order to determine further information about the condition of a subject's circulatory system.

Co-pending application EP 23315495.4 of the same applicant discloses to determine orientation data relating to the expected orientation of a prosthetic cardiac valve with respect to the native cardiac valve on the basis of input data to estimate commissural alignment of an implanted heart valve.

US9693830 B2 discloses to predict an amount of blood flow obstruction through the LVOT of the patient's heart that would occur if the prosthetic valve was to be placed at a designated position in the structure of interest on the basis of a model of an anatomical region of interest and valve models.

EP4084013 A1 discloses methods and systems for assessing an area of interest in the circulatory system of a subject. A temporal sequence of ultrasound image frames of the area of interest and one or more trained models are obtained. The temporal sequence of ultrasound image frames put into the one or more trained models and the temporal sequence of ultrasound image frames is analysed, using the one or more trained models, to produce output data characterising a condition of the area of interest.

It is an object of the present invention to overcome the drawbacks of the prior art. In particular, the system and the method according to the present invention shall provide a reliable risk assessment for paravalvular leaks after a valve replacement.

These and other objects are solved with systems, methods and a computer program according to the independent claims.

According to a first aspect of the invention, a system for automatically assessing a risk of paravalvular leaks after a clinical intervention for implanting a prosthetic cardiac valve in a patient is provided. The system comprises an input interface for obtaining data and a computing unit.

In particular, the system is suitable for automatically assessing a risk of paravalvular leaks after a clinical intervention for implanting a prosthetic aortic valve.

The computing unit is adapted to obtain first input data acquired before the start of the clinical intervention via the input interface. The first input data are related to a specific patient.

The first input data comprise in particular computed tomography (CT) image data of a patient and comprise image data of a region of interest including the aortic root.

The first input data may comprise an entire CT image, or one or more regions obtained from CT images. CT images may provide information, which can be derived from CT images, such as a calcification degree or annular information.

Said computing unit preferably is further adapted to pre-analyse the first input data.

For preliminary analysis of the first input data, an image interpretation tool may be used.

For pre-analysing the first input data, the computing unit may be adapted to enhance the originally obtained first input data, such as to enhance the image quality, to highlight and/or to suppress parts of the image data as a result of the preliminary analysis.

Alternatively, an operator may enhance the first input data.

The computing unit may be adapted to cut or extract a region of interest from the first data.

Generally, the first trained model may be trained such that for example a high risk may be detected directly from the CT images by the AI.

Said computing unit is further adapted to classify the patient into one of two main classes of a likelihood of paravalvular leaks on basis of the first input data. In this context, first input data include enhanced first input data.

A first main class may characterize a low risk for a paravalvular leak after implantation and a second main class may characterize a high risk for a paravalvular leak after implantation.

The first main class may imply that it is possible to choose one of given prosthetic heart valves which is suitable for the specific region of interest, and for which no paravalvular leak is expected.

An assessment of a higher risk (i.e. a classification in the second main class) may give reason for a higher attention of a surgeon, for a further analysis and/or for providing and/or analysing further data.

The first input data may hence be the basis for a first differentiation between a lower and a higher risk for a paravalvular leak.

The system may be adapted to classify the patient purely on basis of CT images, which characterize the status of the region of interest prior to the clinical intervention and, which may include the functionality of the native valve, the geometric arrangement of vessels and properties of the surrounding tissue. This first classification focusses on the region of interest, without taking into account secondary aspects like age, gender, case history or overall condition of the patient.

Thus, the classification provides for a first estimation of the suitability of the patient for the clinical intervention, independent of other possible risk factors.

For classifying the patient into one of the two main classes, the computing unit may be adapted to input first input data into one or more first trained models and to analyse the first input data using the one or more trained first models.

In particular, the computing unit may be adapted to access one or more trained first models, preferably acquired prior to the clinical intervention. The first models may be stored in the system.

The first model may output a respective class or a risk probability indicating whether the input patient belongs to high-risk group.

The computer unit may be adapted to compare the risk probability provided by the first model with a predefined threshold. If the risk probability is bigger than the threshold, then the patient is classified as belonging to a high-risk class, otherwise to a low-risk class.

According to a further aspect a system, preferably as described above, comprises an input interface for obtaining data and a computing unit. The input interface and the computing unit may be the same as described above or may be a separate interface or, unit. The computing unit is adapted to train at least one first model by obtaining, for each of a plurality of training patients, one or more first training input data of that training patient, via the input interface. The first input data comprise CT image data and comprise image data of a region of interest including the aortic root.

For training the at least one first model, the computing unit is further adapted to obtain, for each of the plurality of training patients, ground truth data characterising one of two main classes of likelihood of paravalvular leaks for that training patient.

The computing unit is further adapted to train the at least one first model based on the obtained first training input data and the corresponding ground truth data, such that the at least one first model is trained to produce output data concerning one of two main classes of likelihood of paravalvular leaks for any given patient based on first input data of that patient.

Alternatively, the computing unit may be adapted to obtain, for each of the plurality of training patients, ground truth data characterising a risk probability indicating whether the training patient belongs to a high-risk group. The computing unit may be further adapted to train the at least one first model based on the obtained first training input data and the corresponding ground truth data, such that the at least one first model is trained to produce output data concerning a risk probability for any given patient based on first input data of that patient.

The CT image data may comprise image data comprising data of a region around the implantation site, in particular around the aortic root and/or the sinotubular junction.

The CT image data may particularly comprise image data comprising data of native valve tissue and surrounding structures. These CT image data are suitable to be checked for calcification.

The CT image data particularly may comprise image data comprising data of coronary arteries. These image data are suitable to ensure that there is no impingement on the coronary ostia.

The CT image data particularly may comprise image data comprising data of the ascending aorta. These image data are suitable to be checked for abnormalities, such as dilation or dissection.

The CT image data particularly may comprise image data comprising data nearby structures. These image data are suitable to be checked for possible complications.

The data may represent a 3D region and/or may represent 2D slices.

Preferably, the data include data of regions with a distance of 100% to 500% of the diameter of the aorta in the region of the aortic root from the aortic.root, in particular up to 2-5cm_from the aortic root.

According to a further aspect of the invention, a system for automatically assessing a risk of paravalvular leaks after a clinical intervention for implanting a prosthetic cardiac valve in a patient, preferably as described above, comprises an input interface for obtaining data and a computing unit. The input interface and the computing unit may be the same as described above or may be a separate interface or unit.

The computing unit is adapted to obtain one of two predetermined main classes of likelihood of paravalvular leaks with respect to the patient, in particular determined by a system as described above.

The computing unit is further adapted to obtain second input data collected during the clinical intervention via the input interface. The second input data comprise data representing a temporal sequence of image frames of the area of interest, preferably fluoroscopy image frames.

The computing unit may be adapted to obtain the second input data during the clinical intervention.

The second input data are related to the specific patient.

The second input data may comprise a temporal sequence of image frames of the area of interest, instant single frames or several continuous frames.

The frames may show an entire image or a subregion of an image.

The area of interest may comprise regions with a distance of up to the largest diameter of the heart from the aortic root, in particular 3-10cm from the aortic root.

The data representing a temporal sequence of image frames of the area of interest may be collected during the surgical intervention of placing the prosthetic cardiac valve.

The computing unit is further adapted to determine a preliminary likelihood of paravalvular leaks on the basis of the second input data and the predetermined main class of likelihood of paravalvular leaks.

The computing unit may be adapted to determine the preliminary likelihood of paravalvular leaks during the clinical intervention.

The preliminary likelihood of paravalvular leaks may be determined during the placement of a prosthetic cardiac valve, in particular, before the prosthetic cardiac valve has reached its final position.

This allows a surgeon to react based on an intermediate determined preliminary likelihood of paravalvular leaks and to adapt the placement procedure, eventually to change the orientation and/or the position of the prosthetic valve or even to change the prosthetic valve.

For determining the preliminary likelihood of paravalvular leaks, the computing unit may be adapted to input the second input data and the predetermined main class of likelihood of paravalvular leaks into one or more trained second models.

The computing unit may be adapted to analyse the second input data and the predetermined main class of likelihood of paravalvular leaks using the one or more trained second models.

In particular, the computing unit may be adapted to access the one or more trained second models, preferably acquired, prior to or during the clinical intervention. The trained second models may be stored in the system.

The second trained model may be stored on the same storing device as the first trained model.

The second trained model may be accessed by the same computing unit and in the same way as the first trained model.

According to a further aspect of the invention, a system for automatically assessing a risk of paravalvular leaks after a clinical intervention for implanting a prosthetic cardiac valve in a patient, preferably as described above, comprises an input interface for obtaining data and a computing unit. The input interface and the computing unit may be the same as described above or may be a separate interface or unit.

The computing unit is adapted to train at least one second model by obtaining, for each of a plurality of training patients, one or more second training input data and a training main class of a likelihood of paravalvular leaks for the respective training patient. The second input data comprise data representing a temporal sequence of image frames of the area of interest, preferably fluoroscopy image frames.

The computing unit is further adapted to train a second model by obtaining, for each of the plurality of training patients, ground truth data characterising the preliminary likelihood of PVL for that training patient.

The computing unit is further adapted to train a second model by training the second model based on the obtained second training input data, the training main class of likelihood of paravalvular leaks and the corresponding ground truth data, such that the second model is trained to produce output data concerning the preliminary likelihood of paravalvular leaks for any given patient, based on second input data and a main class of likelihood of paravalvular leaks for that patient.

For machine learning and training in this context deep learning methods, such as Convolutional Neural Networks '(CNN), Recurrent Neural Networks (RNN) may be used. Alternatively, classical machine learning models, such as random forests or support vector machines (SVM) may be used. Also, combinations of multiple methods may be applied.

The data representing the temporal sequence of fluoroscopy image frames may cover a duration longer than or equal to one period of a periodic event associated with the circulatory system such as e.g. a heart beat.

The data representing the temporal sequence of fluoroscopy image frames may represent a 3D region and/or may represent 2D slices.

The computing unit of a system as described above may be adapted to obtain third input data which may be collected during and/or after the clinical intervention via the input interface. The third input data are related to the specific patient.

The computing unit may be adapted to obtain the third input data during and/or after the clinical intervention.

The third input data comprise at least one of
- data representing a temporal sequence of fluoroscopy image frames of the area of interest including the implanted valve,
- electrocardiogram (ECG) data,
- pressure gradient data,
- CT scan data.

The computing unit may be adapted to determine a likelihood of short-term and/or long-term paravalvular leaks on the basis of the third input data, and in particular considering also on the main class of likelihood of paravalvular leaks and/or the preliminary likelihood of paravalvular leaks.

ECG data are typically monitored and/or recorded before, during, and after the implantation procedure, for example a TAVI procedure. Continuous ECG monitoring is crucial to assess the patient's cardiac rhythm and detect any changes throughout the intervention.

The duration of ECG monitoring varies but is often continuous during the entire implantation procedure. It starts before the intervention to establish a baseline and continues throughout the implantation process, allowing real-time assessment of cardiac rhythm and potential changes.

Implantation procedures, such as TAVI procedures, can sometimes trigger arrhythmias due to catheter manipulation and/or the deployment of the prosthetic valve. ECG can be used for monitoring for arrhythmias.

Pressure gradient measurements are typically obtained before, during, and after the implantation procedure, such as a TAVI procedure. Key time points include baseline measurements before the intervention, during valve deployment, and post-implantation to assess the immediate impact of the prosthetic valve on hemodynamic.

CT scan data may be of the same kind as computed tomography (CT) image data of the patient acquired before the start of the clinical intervention via the input interface. CT scan data may be monitored and/or recorded before, during, and after the implantation procedure.

Even after a successful positioning of the prosthetic valve there may still be a risk of, eventually later arising, paravalvular leaks. If this risk can be identified, there is a chance of reducing or preventing short-term and/or long-term paravalvular leaks by taking appropriate measures.

For determining the likelihood of short-term and/or long-term paravalvular leaks, the computing unit may be adapted to input third input data into one or more trained third models and to analyse the third input data using the one or more trained third models.

The computing unit may be adapted to access one or more trained third models, preferably prior, during and/or after to the clinical intervention. The third models may be stored in the system or externally.

The third models may be obtained by the system prior, during and/or after to the clinical intervention.

The computing unit may be adapted to train at least one third model by obtaining, for each of a plurality of training patients, one or more third training input data, in particular a main class of likelihood of paravalvular leaks and/or a preliminary likelihood of paravalvular leaks, and a training likelihood of short-term and/or long-term paravalvular leaks for the respective training patient. The third input data comprise at least one of
- data representing a temporal sequence of fluoroscopy image frames of the area of interest including the implanted valve,
- ECG data,
- pressure gradient data and
- CT scan data.

The computing unit may be further adapted to train the at least one third model by obtaining, for each of the plurality of training patients, ground truth data characterising the likelihood of short-term and/or long-term paravalvular leaks for that training patient.

The computing unit may be further adapted to train the third model based on the obtained third training input data, in particular the main class of likelihood of paravalvular leaks and/or the preliminary likelihood of paravalvular leaks, and the corresponding ground truth data, such that the third model is trained to produce output data concerning the likelihood of short-term and/or long-term paravalvular leaks for any given patient, based on the third input data and in particular on the basis of the main class of likelihood of paravalvular leaks and/or the preliminary likelihood of paravalvular leaks for that patient.

The classification of the patient into one of two main classes of likelihood of paravalvular leaks and/or the determination of the preliminary likelihood of paravalvular leaks as described above may be purely based on the characteristics and the behaviour of the region of interest before and during the clinical intervention.

However, other parameter may also be taken into account, especially for the determination of the preliminary likelihood of paravalvular leaks.

The second input data and/or first input data may comprise patient data relating to characteristics of the patient.

Patient data relating to characteristics of the patient typically are independent of the surgical intervention and may be collected prior to the surgical intervention.

Patient data relating to characteristics of the patient may comprise at least one of gender, age, weight, height, abdominal girth, calcification degree, such as Aortic calcification (AVC) score, annular ellipticity of the valve annulus, smoking status, allergies, cholesterol levels, diabetes status, family history, case history.

The first and/or second input data further may comprise at least one of prosthetic valve data relating to characteristics of the prosthetic valve data to be implanted and delivery tool data relating to characteristics of the delivery tool used during the clinical intervention.

Specific prosthetic valves and delivery tools may be more or less suitable for the clinical intervention for a specific patient, which may have an impact on the risk for paravalvular leaks.

The first and/or second input data further may comprise at least one of pressure gradient data, ultrasound (US) image data, fused image data, ECG data, projection angle.

In fact, often the anatomy information is not very visible on a fluoroscopy image. Hence, some prior information may be needed. Fused image data could be created from CT images being projected to fluoroscopic images, or from prior anatomy information, for example obtained from CT, and then projected on fluoroscopy.

In Transcatheter Aortic Valve Implantation (TAVI) procedures, the projection angle refers to the angle at which X-ray images are acquired during fluoroscopy.

Before the TAVI intervention, CT is used to determine the optimal annular plane, which is the plane perpendicular to the long axis of the aorta and passing through the aortic annulus. This plane is used as a reference for determining the projection angle.

These data may be collected and/or obtained before or during the clinical intervention. They may provide for further characterizing the region of interest and/or the overall status of the patient.

In particular, the second input data may include all available pre-operationally and intra-operationally derived information.

The computing unit of a system as described above may be adapted for providing output data to an output interface.

The output data may include a main class of likelihood of paravalvular leaks, a preliminary likelihood of paravalvular leaks and/or a likelihood of short-term and/or long-term paravalvular leaks.

The system may further comprise an output interface.

The output interface may be an output device for representing output data, selected from the group consisting of a monitor, in particular a 3D monitor, a display, a virtual reality or an augmented reality interface, a warning indicator,
a visual pointer.

The output device may an output device which is used by a surgeon during the surgical intervention also for other purposes.

A system as described above may be part of a system for at least partly automatically performing the clinical intervention and/or of a system for monitoring the patient before, during and/or after the clinical intervention.

Another aspect of the invention relates to a method for automatically assessing a risk of paravalvular leaks after a clinical intervention for implanting a prosthetic cardiac valve in a patient. The method may use a system comprising an input interface and a computing unit, preferably as described above.

First input data are obtained before the start of the clinical intervention via an input interface. The first input data comprise image data comprising image data of a region of interest including the aortic root and may be first input data as described above, in particular CT image data.

The method may comprise the step of pre-analysing the CT image data as described above, as explained above.

Data concerning a region of interest may be cut or extracted for the first input data.

The method further comprises the step of classifying the patient into one of two main classes of likelihood of paravalvular leaks on the basis of the first input data.

The method may comprise the further steps of inputting first input data into one or more first trained models and analysing the first input data using the one or more trained first models.

In particular, for this purpose one or more trained first models are accessed, preferably prior to the clinical intervention.

The trained first models may be stored on the system or externally.

The one or more trained first models may be obtained by the system prior to the clinical intervention.

A method for training at least one first model may use a system comprising an input interface and a computing unit, preferably as described above.

The method may be part of the method for automatically assessing a risk of paravalvular leaks after a clinical intervention as described above.

The method for training at least one first model comprises the following steps.

For each of a plurality of training patients, one or more first training input data of that training patient, are obtained. The first input data comprise CT image data comprising image data of a region of interest including the aortic root.

For each of the plurality of training patients, ground truth data characterising one of two main classes of likelihood of paravalvular leaks for that training patient are obtained.

The first model is trained based on the obtained first training input data and the corresponding ground truth data, such that the first model is trained to produce output data concerning one of two main classes of likelihood of paravalvular leaks for any given patient, based on first input data of that patient.

The method for training a first model may be part of the method for automatically assessing a risk of paravalvular leaks after a clinical intervention for implanting a prosthetic cardiac valve in a patient as described above.

The CT image data are preferably CT image data as described above.

A method for automatically assessing a risk of paravalvular leaks after a clinical intervention for implanting a prosthetic cardiac valve in a patient uses a system comprising an input interface and a computing unit, preferably as described above.

The method may be carried out following the method for automatically assessing a risk of paravalvular leaks after a clinical intervention as described above.

Second input data are obtained during the clinical intervention via the input interface. The second input data comprise a temporal sequence of image frames of the area of interest and may be second input data as described above.

A preliminary likelihood of paravalvular leaks is determined on the basis of the second input data and a predetermined main class of likelihood of paravalvular leaks.

The second input data and the predetermined main class of likelihood of paravalvular leaks may be put into one or more trained second models and the second input data and the predetermined main class of likelihood of paravalvular leaks may be analysed using the one or more trained second models.

The one or more trained second models may be stored on the system or externally.

In particular, for this purpose one or more trained second models are accessed, preferably prior to or during the clinical intervention.

The one or more trained second models may be obtained prior to or during the clinical intervention.

A method for training at least one second model may use a system comprising an input interface and a computing unit, preferably as described above.

For each of a plurality of training patients, one or more training second input data and a training main class of likelihood of paravalvular leaks for that training patient are obtained. The second input data comprise a temporal sequence of fluoroscopy image frames of the area of interest.

For each of the plurality of training patients, ground truth data characterising the preliminary likelihood of PVL are obtained.

The at least one second model is trained based on the obtained second training input data, the training main class of likelihood of paravalvular leaks and the corresponding ground truth data, such that the second model is trained to produce output data concerning the preliminary likelihood of paravalvular leaks for any given patient, based on second input data and a main class of likelihood of paravalvular leaks for that patient.

The method for training at least one second model may be part of the method for automatically assessing a risk of paravalvular leaks after a clinical intervention including the determination of a preliminary likelihood of paravalvular leaks on the basis of the second input data and a predetermined main class of likelihood of paravalvular leaks as described above.

The temporal sequence of fluoroscopy image frames may be a temporal sequence of fluoroscopy image frames as described above.

The methods as described above may comprise the further steps of receiving third input data during and/or after the clinical intervention via the input interface and determining a likelihood of short-term and/or long-term paravalvular leaks on the basis of the third input data, and in particular also on the basis of the main class of likelihood of paravalvular leaks and/or the preliminary likelihood of paravalvular leaks.

The method steps may be carried out during and/or after the clinical intervention.

The third input data are third input data as described above.

The method may comprise the step of inputting the third input data into one or more trained third models.

The third input data may be analysed using the one or more trained third models.

One or more trained third models may be accessed, preferably during and/or after to the clinical intervention.

One or more trained third models may be obtained by the system prior, during and/or after to the clinical intervention.

A method for training at least one third model may use a system comprising an input interface and a computing unit, preferably as described above.

For each of a plurality of training patient, one or more training third input data and, in particular, a training main class of a likelihood of paravalvular leaks and/or a training preliminary likelihood of paravalvular leaks may be obtained.

For each of the plurality of training patients, ground truth data characterising the likelihood of short-term and/or long-term paravalvular leaks for that training patient may be obtained.

The at least one third model may be trained based on the obtained third training input data, in particular the training main class of likelihood of paravalvular leaks and/or a training preliminary likelihood of paravalvular leaks, and the corresponding ground truth data, such that the third model is trained to produce output data concerning the likelihood of short-term and/or long-term paravalvular leaks for any given patient, based on the third input data and, in particular, a main class of likelihood of paravalvular leaks and/or a likelihood paravalvular leaks, for that patient.

The method for training a third model may be part of any of the methods as described above.

The first and/or the second input data further may comprise first and/or the second input data as explained above.

According to another aspect of the invention, a computer program comprises program code for carrying out the steps of the method(s) as described above when the program is executed on a computer of a system or a computer being connected to a system as described above.

According to still another aspect of the invention, a computer program product which can be loaded directly into an internal memory of a digital computer, for example being part of a system as described above, comprises software code portions executing the method steps as described above when the program is running on the digital computer being part of a system or being connected to a system as described above.

The invention is explained in the following by means of exemplary embodiments and the accompanying drawing, in which:
- Fig. 1: is a schematic presentation of a system according to the invention;
- Fig. 2: is a presentation of an example of first input data;
- Fig. 3: is a schematic presentation of a first example of a method according to the invention;
- Fig. 4: is a schematic presentation of a second example of a method according to the invention.

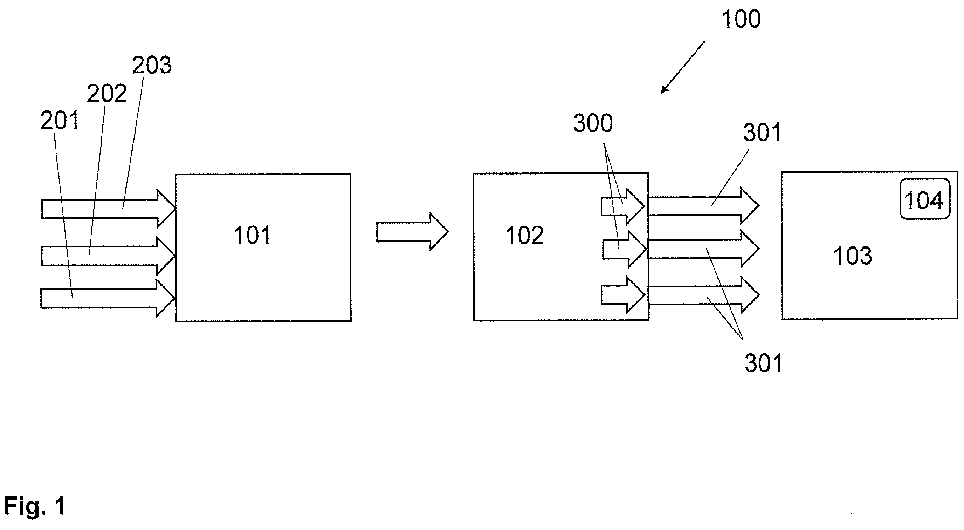

Figure 1 shows a system 100 for automatically assessing a risk of paravalvular leaks after a clinical intervention for implanting a prosthetic cardiac valve in a patient. The system 100 comprises an input interface 101 for obtaining data and a computing unit'102.

Said computing unit 102 is adapted to obtain first input data 201 acquired before the start of the clinical intervention, second input data 202 acquired during the clinical intervention and third input data 203 acquired during and/or after the clinical intervention via the input interface 101.

The computer unit is adapted to determine risk parameters 300, such as to classify the patient into one of two main classes of likelihood of paravalvular leaks on the basis of the first input data 201 and to determine a preliminary likelihood of paravalvular leaks on the basis of the second input data 202 and the predetermined main class of likelihood of paravalvular leaks.

Said computing unit 102 is further adapted for providing output data 301 about the risk parameters 300, in particular concerning a main class of likelihood of paravalvular leaks and/or a preliminary likelihood of paravalvular leaks, to an output interface 103.

The output interface 103 comprises an output device 104 for representing output data 301.

First, second and/or third input data may be provided by an external device, such as a measurement device, retrieved from a storage device or provided by an operator.

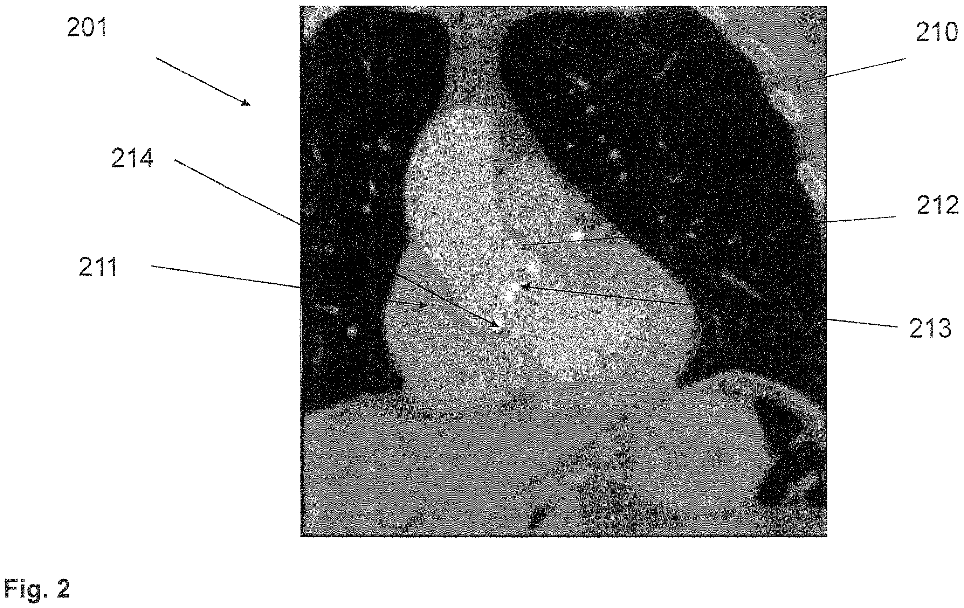

Figure 2 shows a 2D-presentation of an example of first input data 201. The first input data 201 comprise data of a CT image 210 of a patient and comprise image data of a heart 211 of a patient. The region of interest 212 is a section of the CT image 210 and includes the aortic root 213.

As can be seen on the CT image 210 the region of interest 212 comprises calcifications 214, which may depend on the position and on the amount imply a higher risk.

The first input data 201 may comprise data representing the patient before implantation of a prosthetic valve and hence may not comprise data concerning the prosthetic valve.

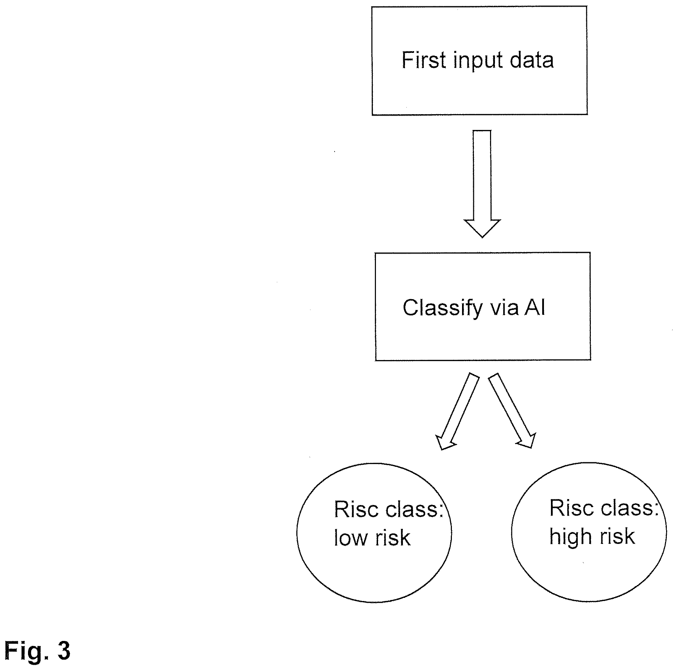

Figure 3 schematically shows a schematic presentation of a first example of a method according to the invention.

The method comprises the step of obtaining first input data 201 related to a specific patient before the start of the clinical intervention via the input interface 101 (see figure 1).

The first input data 201 comprise CT image data of the patient including image data of a region of interest including the aortic root.

In a further step, the patient is classified into one of two main classes of likelihood of paravalvular leaks, namely a first risk class characterizing a low risk and a second risk class characterizing a high risk, on the basis of the first input data 301.

In this example the patient is classified via an AI process using one or more first trained models.

A classification model may be a type of machine learning model designed to categorize or classify input data into predefined classes or categories. The designed model may learn patterns and/or relationships within the data that allow the model to make accurate predictions about low risk, high risk or a probability of risk. Various algorithms could be used to build classification models, such as Decision Trees, Support Vector Machines (SVM), Naive Bayes, Logistic Regression, and Neural Networks .

The first trained models may be stored on the system 100 (see figure 1) and/or may be provided by a training routine executed on the same system 100 (see figure 1) or on a separate device.

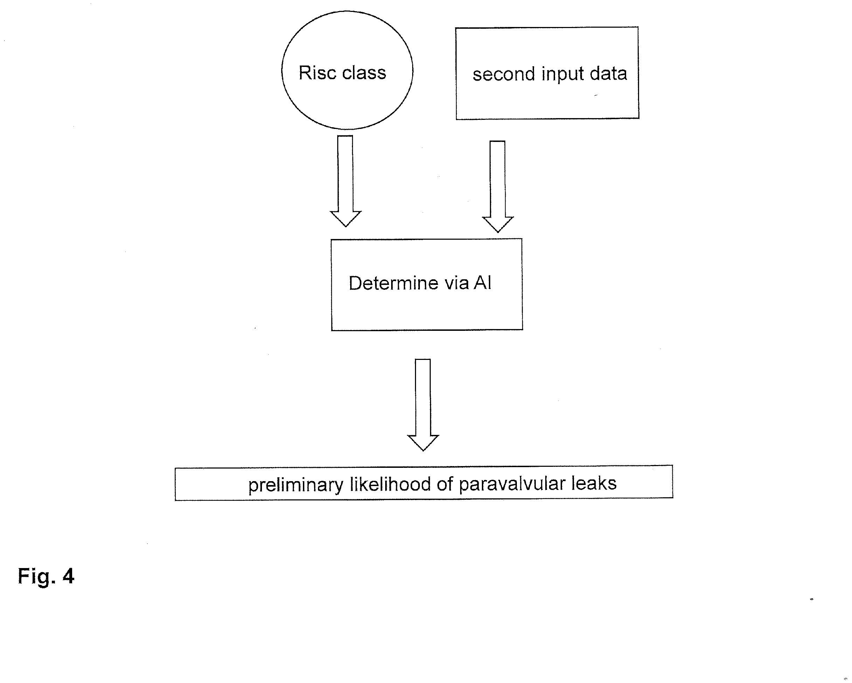

Figure 4 is a schematic presentation of a second example of a method according to the invention.

The method comprises the step of obtaining second input data 202 related to a specific patient during a clinical intervention via the input interface 101 (see figure 1).

The method comprises the step of obtaining a predetermined main class of likelihood of paravalvular leaks.

In a further step, a preliminary likelihood of paravalvular leaks after the clinical intervention is determined on the basis of the second input data 202 and a predetermined main class of likelihood of paravalvular leaks.

The predetermined main class may be provided by the system 100 (see figure 1) or retrieved from an external source.

The predetermined main class is one of two main classes and may have been determined in a first part of the method or in a separate method, as shown in figure 3.

In this example the preliminary likelihood is determined via an AI process using one or more second trained models.

The second trained models may be stored on the system 100 (see figure 1) and/or may be provided by a training routine executed on the system 100 (see figure 1) or on a separate device.

Convolutional Neural Network (CNN) may be used for PVL classification within the first model as CNNs are well-suited for image-related tasks, making them suitable for analysing CT scans. In particular, a simplified CNN architecture could include convolutional layers to capture spatial features, pooling layers for down-sampling, and fully connected layers for classification. The model may be trained under a binary classification setup by using cross-entropy as loss function.

The dataset may include images of patients with prosthetic heart valves.

Second input data 202 may in particular comprise intra-operational image data, for example fluoroscopy data. These data may show the region of interest including the prosthetic valve.

Some preprocessing steps may have been applied such as normalization, resizing and ROI extraction which is defined as the 3D region between root and sinotubular junction. Necessary data augmentation techniques could also be used such as random rotations, flips, and zooms to artificially increase the diversity of the training set.

For the second model, a hybrid model may be used combining a convolutional neural network (CNN) for image analysis with a traditional machine learning model (e.g., logistic regression) for structured data. A traditional machine learning algorithm (e.g., logistic regression) could be applied to process the structured data (prosthesis type, calcification degree, patient age, ellipticity of the aortic valve outlet, etc). A CNN is to process and analyse the fluoroscopy images. Both paths (structured data and image data) are combined using an ensemble approach or a concatenated layer to make the final PVL prediction.

The second trained models may be applied during the surgical intervention. Thus, the preliminary likelihood of paravalvular leaks may be determined during the placement of the prosthetic valve. Eventually, the prosthetic valve may be replaced, the second trained models may be applied to new data including the replaced valve and a new preliminary likelihood of paravalvular leaks may be determined.

The second trained model may also be applied after the clinical intervention.

## Claims

1. A system (100) for automatically assessing a risk of paravalvular leaks after a clinical intervention for implanting a prosthetic cardiac valve in a patient, the system (100) comprising
- an input interface (101) for obtaining data,
- a computing unit (102),
wherein said computing unit (102) is adapted
o to obtain first input data (201) acquired before the start of the clinical intervention via the input interface (101), the first input data comprising CT image data of a patient and comprising image data of a region of interest, preferably including the aortic root,
o preferably to pre-analyse the first input data,
o to classify the patient into one of two main classes of likelihood of paravalvular leaks on basis of the first input data.

2. The system according to claim 1, wherein for classifying the patient into one of two main classes, the computing unit (102) is adapted
- to input first input data (201) into one or more first trained models,
- to analyse the first input data (201) using the one or more trained first models.

3. A system, preferably according to claim 1 or 2, the system (100) comprising
- an input interface (101) for obtaining data,
- a computing unit (102),
wherein the computing unit (102) is adapted to train at least one first model by
- obtaining, for each of a plurality of training patients, one or more first training input data of that training patient, via the input interface (101), the first input data comprising CT image data comprising image data of a region of interest including the aortic root
- obtaining, for each of the plurality of training patients, ground truth data characterising one of two main classes of likelihood of paravalvular leaks for that training patient; and
- training the first model based on the obtained first training input data and the corresponding ground truth data, such that the first model is trained to produce output data concerning one of two main classes of likelihood of paravalvular leaks for any given patient, based on first input data of that patient.

4. The system according to one of the preceding claims, wherein the CT image data comprise data about a region around the aortic root.

5. A system for automatically assessing a risk of paravalvular leaks after a clinical intervention for implanting a prosthetic cardiac valve (2) in a patient, preferably according to one of claims 1-4, the system (100) comprising
- an input interface (101) for obtaining data,
- a computing unit (102),
wherein the computing unit (102) is adapted
- to obtain one of two predetermined main classes of likelihood of paravalvular leaks,
- to obtain second input data (202) collected during the clinical intervention via the input interface (101), the second input data (202) comprising data representing a temporal sequence of image frames of the area of interest, preferably fluoroscopy image frames, and
- to determine a preliminary likelihood of paravalvular leaks on the basis of the second input data and the predetermined main class of likelihood of paravalvular leaks.

6. The system according to claim 5, wherein for determining the preliminary likelihood of paravalvular leaks, the computing unit (102) is adapted
- to input second input data (202) and the predetermined main class of likelihood of paravalvular leaks into one or more trained second models,
- to analyse the second input data (202) and the predetermined main class of likelihood of paravalvular leaks using the one or more trained second models.

7. The system, preferably according to claim 5 or 6, the system (100) comprising
- an input interface (101) for obtaining data,
- a computing unit (102),
wherein the computing unit (102) is adapted to train at least one second model by
- obtaining, for each of a plurality of training patients, one or more second training input data and a training main class of a likelihood of paravalvular leaks for the respective training patient, the second input data comprising
o data representing a temporal sequence of image frames of the area of interest, preferably fluoroscopy image frames;
- obtaining, for each of the plurality of training patients, ground truth data characterising the preliminary likelihood of PVL for that training patient; and
- training the second model based on the obtained second training input data, the training main class of likelihood of paravalvular leaks and the corresponding ground truth data, such that the second model is trained to produce output data concerning the preliminary likelihood of paravalvular leaks for any given patient, based on second input data and a main class of likelihood of paravalvular leaks for that patient.

8. The system according to one of claims 5-7, wherein the data representing the temporal sequence of fluoroscopy image frames cover a duration longer than or equal to one period of a periodic event associated with the circulatory system.

9. The system according to one of the preceding claims,
wherein the computing unit (102) is adapted
- to obtain third input data (203) during and/or after the clinical intervention via the input interface (101), the third input data (203) comprising at least one of
o a temporal sequence of fluoroscopy image frames of the area of interest including the implanted valve,
o ECG data,
o Pressure gradient data,
o CT scan data,
- to determine a likelihood of short-term and/or long-term paravalvular leaks on the basis of the third input data (203), and in particular on the basis of the main class of likelihood of paravalvular leaks and/or the preliminary likelihood of paravalvular leaks.

10. The system according to claim 9, wherein for determining a likelihood of short-term and/or long-term paravalvular leaks the computing unit (102) is adapted
- to input third input data into one or more trained third models,
- to analyse the third input data using the one or more trained third models.

11. The system according to one of the preceding claims,
wherein the second input data and/or first input data comprise patient data relating to characteristics of the patient.

12. The system according to one of the preceding claims,
wherein the first and/or second input data further comprise at least one of
- prosthetic valve data relating to characteristics of the prosthetic valve data to be implanted,
- delivery tool data relating to characteristics of the delivery tool, used during the clinical intervention,
- pressure gradient data,
- US image data,
- Fused image data,
- ECG data,
- projection angle.

13. A system according to any of the preceding claims, wherein the computing unit (102) is adapted for providing output data (301), in particular concerning a main class of likelihood of paravalvular leaks and/or a preliminary likelihood of paravalvular leaks, to an output interface (103), in particular the system comprising an output interface (103) .

14. A system according to claim 13, wherein the output interface (103) comprises an output device (104) for representing output data (301), selected from the group consisting of
- a monitor, in particular a 3D monitor,
- a display,
- a virtual reality or an augmented reality interface,
- a warning indicator,
- a visual pointer.

15. A method for automatically assessing a risk of paravalvular leaks after a clinical intervention for implanting a prosthetic cardiac valve (2) in a patient, preferably using a system according to one of claims 1-14, comprising the steps of
- obtaining first input data (301) before the start of the clinical intervention via the input interface (101), the first input data (301), comprising image data, in particular including the aortic root, preferably comprising CT image data,
- classifying the patient into one of two main classes of likelihood of paravalvular leaks on the basis of the first input data.

16. The method according to claim 15, comprising the further steps of
- inputting first input data into one or more first trained models,
- analysing the first input data using the one or more trained first models.

17. A method for training at least one first model, preferably according to claim 15 or 16, comprising the steps of
- obtaining, for each of a plurality of training patients, one or more first training input data of that training patient, the first input data comprising CT image data comprising image data of a region of interest including the aortic root
- obtaining, for each of the plurality of training patients, ground truth data characterising one of two main classes of likelihood of paravalvular leaks for that training patient; and
- training the first model based on the obtained training first input data and the corresponding ground truth data, such that the first model is trained to produce output data concerning one of two main classes of likelihood of paravalvular leaks for any given patient, based on first input data of that patient.

18. A method according to at least one of claims 15-17, wherein the CT image data comprise image data of a region including the aortic root, preferably comprise data about a 3D region around the aortic root, preferably including the aortic root and the sinotubular junction.

19. A method, preferably according to one of claims 15-18, for automatically assessing a risk of paravalvular leaks after a clinical intervention for implanting a prosthetic cardiac valve (2) in a patient, preferably using a system according to one of claims 1-14, comprising the steps of
- obtaining second input data during the clinical intervention via the input interface (101), the second input data comprising a temporal sequence of image frames of the area of interest, preferably fluoroscopy image frames and
- determining a preliminary likelihood of paravalvular leaks on the basis of the second input data and a predetermined main class of likelihood of paravalvular leaks.

20. A method according to claim 19, comprising the further steps of
- inputting second input data and the predetermined main class of likelihood of paravalvular leaks into one or more trained second models,
- analysing the second input data and the predetermined main class of likelihood of paravalvular leaks using the one or more trained second models.

21. A method for training at least one second model, preferably according to one of claims 19 or 20, comprising the steps of
- obtaining, for each of a plurality of training patient, one or more training second input data and a training main class of preliminary likelihood of paravalvular leaks for that training patient, the second input data comprising a temporal sequence of fluoroscopy image frames of the area of interest;
- obtaining, for each of the plurality of training patients, ground truth data characterising the preliminary likelihood of PVL; and
- training the at least one second model based on the obtained second training input data, the training main class of preliminary likelihood of paravalvular leaks and the corresponding ground truth data, such that the second model is trained to produce output data concerning the preliminary likelihood of paravalvular leaks for any given patient, based on second input data and a main class of likelihood of paravalvular leaks for that patient.

22. Method according to one of claims 19-21, wherein the temporal sequence of fluoroscopy image frames is three-dimensional and/or
the temporal sequence of fluoroscopy image frames has a duration longer than or equal to one period of a periodic event associated with the circulatory system.

23. Method according to one of claims 15-21, comprising the further steps of
- obtaining third input data (203) during and/or after the clinical intervention via the input interface (101), the third input data comprising at least one of
o a temporal sequence of fluoroscopy image frames of the area of interest including the implanted valve,
o ECG data,
o Pressure gradient data,
o CT scan data,
- determining a likelihood of short-term and/or long-term paravalvular leaks on the basis of the third input data, and in particular on basis of the main class of likelihood of paravalvular leaks and/or the preliminary likelihood of paravalvular leaks.

24. A method according to claim 23, comprising the further steps of
- inputting third input data into one or more trained third models,
- analysing the third input data using the one or more trained third models.

25. A method according to one of claims 15-24, wherein the first and/or second input data further comprise at least one of
- patient data relating to characteristics of the patient;
- prosthetic valve data relating to characteristics of the prosthetic valve data to be implanted,
- delivery tool data relating to characteristics of the delivery tool, used during the clinical intervention,
- pressure gradient data,
- US image data,
- Fused image data,
- ECG data.

26. A computer program comprising program code for carrying out the steps of the method according to any one of the claims 15 to 25 when the program is executed on a computer of a system according to one of claims 1-14.

27. A computer program product which can be loaded directly into an internal memory of a digital computer and which comprises software code portions executing the method steps of at least one of the claims 15 to 25 when the program is running on the digital computer of a system according to one of claims 1-14.
